# EUROPEAN PATENT SPECIFICATION

(11) **EP 3 658 543 B1**
(45) Date of publication and mention of the grant of the patent: **09.12.2026**
(21) Application number: 18752098.6
(22) Date of filing: 20.07.2018
(51) Int. Cl.: C07D 271/06

(54) **PREPARATION OF SUBSTITUTED 3-ARYL-5-TRIFLUOROMETHYL-1,2,4-OXADIAZOLES**
HERSTELLUNG VON SUBSTITUIERTEN 3-ARYL-5-TRIFLUORMETHYL-1,2,4-OXADIAZOLEN
PRÉPARATION DE 3-ARYL-5-TRIFLUOROMÉTHYL-1,2,4-OXADIAZOLES SUBSTITUÉS

(30) Priority: 28.07.2017 EP 17183655
(43) Date of publication of application: 03.06.2020
(73) Proprietor: BASF Agricultural Solutions Deutschland GmbH, 67117 Limburgerhof (DE)
(72) Inventor: GRAMMENOS, Wassilios, 67056 Ludwigshafen (DE); RACK, Michael, 67056 Ludwigshafen (DE); TERTERYAN-SEISER, Violeta, 67056 Ludwigshafen (DE); KORADIN, Christopher, 67056 Ludwigshafen (DE)
(74) Representative: AP-IP
(86) International application number: PCT/EP2018/069712
(87) International publication number: WO 2019/020501

(56) References cited:
- WO-A1-2015/185485
- CHENG-MING CHU ET AL: "Bioisosteric replacement of the pyrazole 3-carboxamide moiety of rimonabant. A novel series of oxadiazoles as CB1 cannabinoid receptor antagonists", ORGANIC & BIOMOLECULAR CHEMISTRY, vol. 6, no. 18, 1 January 2008 (2008-01-01), pages 3399, XP055502922, ISSN: 1477-0520, DOI: 10.1039/b807648k
- ANDERSEN K ET AL: "Oxadiazoles as bioisosteric transformations of carboxylic functionalities. Part I", EUROPEAN JOURNAL OF MEDICINAL CHEMISTRY, EDITIONS SCIENTIFIQUE ELSEVIER, PARIS, FR, vol. 29, no. 5, 1 January 1994 (1994-01-01), pages 393 - 399, XP023870431, ISSN: 0223-5234, [retrieved on 19940101], DOI: 10.1016/0223-5234(94)90065-5
- LIU GANG ET AL: "Discovery of potent, selective, orally bioavailable Stearoyl-CoA desaturase 1 inhibitors", JOURNAL OF MEDICINAL CHEMISTRY, AMERICAN CHEMICAL SOCIETY, vol. 50, no. 13, 1 June 2007 (2007-06-01), pages 3086 - 3100, XP002545150, ISSN: 0022-2623, DOI: 10.1021/JM070219P

## Description

The present invention relates to a process for the preparation of substituted 3-aryl-5-trifluoromethyl-1,2,4-oxadiazoles (compounds I), which can be obtained through reaction of hydroxyamidine type compounds of formula II with trifluoroacetyl halides.

Certain 3-substituted 5-trifluoromethyl-1,2,4-oxadiazoles are known as fungicides i.a. from EP 276432 A2, EP 3165093 A1, EP 3165094 A1, EP 3167716 A1, WO 2015/185485, WO 2017/055469, WO 2017/055473, WO 2017/076739, WO 2017/076740, WO 2017/076742, WO 2017/076757, WO 2017/076935, WO 2017/081309, WO 2017/081310, WO 2017081311, WO 2017081312, WO 2017/085098, WO 2017/085100, WO2017/093019, WO2017/093348, WO 2017/085100, WO2017/093348, WO2017/102006, WO2017/103219, WO2017/103223, WO2017/109044 and WO2017/118689.

The formation of the 1,2,4-oxadiazole ring system in 3-aryl-5-trifluoromethyl-1,2,4-oxadiazoles, which are useful as fungicidally active compounds or as intermediates in the synthesis of fungicides, is described in the references cited above or in WO 2013/008162 by reacting amidoximes of type II with trifluoroacetic anhydride (TFAA) in an organic solvent.

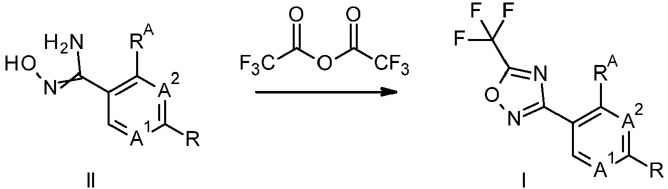

One disadvantage of this reaction lies in the formation of significant amounts of free trifluoroacetic acid (TFA) as a side product. Regulatory restrictions oftentimes require very low residue levels of TFA impurities in commercial agrochemical pesticides. For the sake of receiving a market authorization it is therefore crucial to control and minimize the amount of residual TFA during the process of producing a pesticide.

In contrast to other acids it is particlularly difficult to remove free TFA from reaction mixtures, be it in solution or in mixtures that are in a solid state. TFA has a tendency to form cocrystals or clathrates with other solid reaction components. The removal of TFA is oftentimes cumbersome and requires elaborate additional purification steps (e. g. chromatography, destillation, crystallization, etc.). Furthermore, TFA is very corrosive and there is an interest to reduce the excess amounts of TFA furnished during or after the ring closing reaction, for example during workup. The reaction of compound II with TFAA produces one equivalent TFA. In the course of the oxadiazole ring closing/aromatisation reaction one equivalent of water is formed per equivalent of compound II. The water immediately reacts with another equivalent of TFAA, which in turn results in the formation of two additional equivalents TFA. As a result, at least two equivalents TFAA are necessary for a complete conversion of compounds II and in total at least three equivalents TFA are formed per equivalent of compounds II.

Reducing the number of equivalents of TFAA to the minimum required by theory, i.e. to two equivalents based on the amounts of compound II, does not always provide sufficient yield of the desired product. Typically, an excess of TFAA is used to achieve complete conversion of compounds II to compounds I. Hydrolyzation of excess TFAA under aqueous workup conditions produces even more TFA.

Thus, it was an object of the present invention to overcome the disadvantages of the known processes and to provide an improved and more economical and production plant friendly process.

It was now found that 3-aryl-5-trifluoromethyl-1,2,4-oxadiazoles can be obtained by the use of trifluoroacetyl halides, in particular trifluoroacetyl chloride (TFAC) or trifluoroacetyl fluoride (TFAF). The use of TFAC or TFAF instead of TFAA has several surprising advantages in relation to the preparation of the above-mentioned compounds.

The process of this invention does not require a large excess of trifluoroacetyl halides in order to achieve high yields of the desired oxadiazoles, which is desirable from both, an economical and an ecological point of view. As a consequence, the total organic carbon content in the production waste water is reduced compared to the prior art processes, which makes the process eco-friendly. Unlike the use of TFAA, the use of trifluoroacetic halides results in the formation of a much smaller amount of TFA, which makes such process more production plant friendly and simplifies the workup procedure. As a result the production costs are significantly lower. Furthermore, the process of this invention provides the desired oxadiazoles in high yields and with reduced amounts of undesired side products. The in-situ preparation of compounds of type II enables a process, which proceeds in a smooth and controlled manner, which is very safe, simple, economical, user-friendly and commercially viable.

Accordingly, the present invention relates to a process for preparing compounds of formula I, 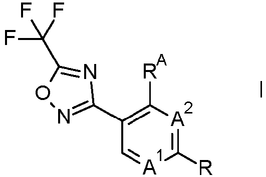 wherein
- A¹: is CH;
- A²: is CH;
- R^{A}: is hydrogen or fluorine;
- R: is -COOH, -COOR¹, -C(=W)NR¹R²;
- W: is O or S;
- R¹: is C₁-C₆-alkyl, C₂-C₆-alkenyl, C₂-C₆-alkynyl, C₁-C₆-alkoxyimino-C₁-C₄-alkyl, C₃-C₁₁-cycloalkyl, C₃-C₈-cycloalkenyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms; and wherein any of the aliphatic or cyclic groups are unsubstituted or substituted with 1, 2, 3 or up to the maximum possible number of identical or different radicals selected from the group consisting of halogen, cyano, C₁-C₆-alkyl and C₁-C₆-alkoxy;
- R²: is hydrogen, formyl, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, C₁-C₆-alkylamino or diC₁-C₆-alkylamino; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy;
the process comprising:
reacting a compound of formula II, 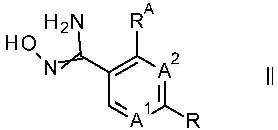
wherein A¹, A², R^{A} and R is as defined above, with a compound of formula IIa, 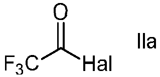
wherein Hal is chlorine or fluorine; and wherein the process is conducted in the presence of an inert organic solvent; and wherein the process is conducted at a temperature from 0°C to 100°C.

The amidoxime compounds II can be obtained from cyano compounds V by treatment with hydroxylamine or a salt thereof, for example the hydrochloride salt, in the presence of a base, preferably triethylamine, in a suitable solvent, such as methanol, at a temperature between 0 °C and 100 °C. For related examples see Kitamura, S. et al Chemi. Pharm. Bull. (2001), 49, 268 WO 2013/066838 or WO 2017/055473:

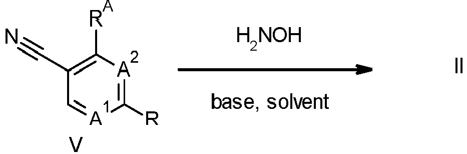

Preferably, the formation of the amidoxime II and its transformation to compounds I takes place in a two-step one-pot reaction without any workup of the amidoxime II.

Compounds of formula III are either commercially available or may be prepared using standard procedures known to a person skilled in the art from readily available starting materials. Such procedures are described in, for example, WO 2013/008162, EP 276432 A2, EP 3165093 A1, EP 3165094 A1, EP 3167716 A1, WO 2015/185485, WO 2017/055469, WO 2017/055473, WO 2017/076739, WO 2017/076740, WO 2017/076742, WO 2017/076757, WO 2017/076935, WO 2017/081309, WO 2017/081310, WO 2017081311, WO 2017081312, WO 2017/085098, WO 2017/085100, WO2017/093019, WO2017/093348, WO2017/102006, WO2017/103219, WO2017/103223, WO2017/109044 and WO2017/118689.

According to a preferred embodiment, compound IIa is trifluoacetyl chloride (TFAC).

In a preferred embodiment, the transformation of a compound of formula II with compounds of formula IIa to produce compounds of formula I is carried out in the presence of an inert organic solvent, wherein the inert organic solvent is not identical with a compound I, II, Ila, III or a base as defined herein.

In a preferred embodiment, said process is carried out in the presence of a base, wherein the base is not identical with a compound I, II, Ila, III or an inert organic solvent as defined herein.

In another preferred embodiment, said process is carried out in the presence of an inert organic solvent, wherein the inert organic solvent is not identical with a compound I, II, IIa, III or a base as defined herein; and in the absence of a base, wherein the base is not identical with a compound I, II, IIa, III or an inert organic solvent as defined herein.

In another preferred embodiment, said process is carried out in the presence of a base, wherein the base is not identical with a compound I, II, IIa, III or an inert organic solvent as defined herein; and in the absence of an inert solvent, wherein the inert organic solvent is not identical with a compound I, II, IIa, III or a base as defined herein.

In another preferred embodiment, said process is carried out in the presence of a base, wherein the base is not identical with a compound I, II, Ila, III or a solvent as defined herein; and in the presence of an inert solvent, wherein the inert organic solvent is not identical with a compound I, II, Ila, III or a base as defined herein.

By "inert organic solvent" is meant an organic solvent which, under the reaction conditions of the process of this invention, does not enter into any appreciable reaction with either the reactants or the products.

In one embodiment, the inert organic solvent is selected from non-halogenated inert organic solvents; preferably from non-halogenated aliphatic hydrocarbons, non-halogenated cycloaliphatic hydrocarbons, non-halogenated aromatic hydrocarbons, halogenated aliphatic hydrocarbons, halogenated aromatic hydrocarbons, amides, ethers, esters, ketones, nitriles and any combination thereof.

Examples of suitable non-halogenated aliphatic hydrocarbons include pentane, hexane, heptane, and the like. Preference is given to saturated aliphatic hydrocarbons having from 5 to 10 carbon atoms.

Examples of suitable non-halogenated cycloaliphatic hydrocarbons include cyclopentane, cyclohexane, cycloheptane, and the like. Preference is given to non-halogenated saturated cycloaliphatic hydrocarbons having from 5 to 10 carbon atoms. Cyclohexane is particularly preferred. Examples of suitable a non-halogenated aromatic hydrocarbons include toluene, o-xylene, m-xylene, p-xylene, ethylbenzene, 2-propylbenzene (cumene), 2-isopropyltoluene (o-cymol), 3-isopropyltoluene (m-cymol), 4-isopropyltoluene (p-cymol), 1,3,5-trimethylbenzene (mesitylene), and the like. Preference is given to toluene, o-xylene, m-xylene, p-xylene, ethylbenzene, 1,3,5-trimethylbenzene (mesitylene), and any combination thereof. Especially preferred among the non-halogenated aromatic hydrocarbons are toluene, o-xylene, m-xylene, p-xylene, and any combination thereof, with toluene being the most preferred.

Examples of suitable halogenated aliphatic hydrocarbons include dichloromethane, chloroform, carbon tetrachloride, 1,2-dichloroethane, 1,1,1,2-tetrachloroethane, 1,1,2,2-tetrachloroethane, 1,1-dichloroethylene, 1,2-dichloroethylene, and the like. Preference is given to dichloromethane and 1,2-dichloroethane and any combination thereof.

Examples of suitable halogenated aromatic hydrocarbons include chlorobenzene, bromobenzene, o-dichlorobenzene, m-dichlorobenzene, α,α,α-trifluorotoluene (benzotrifluoride) and the like and any combination thereof.

Examples of suitable amides include N,N-dimethylformamide, N,N-dimethylacetamide, N,N-diethylacetamide, N-methyl-pyrrolidone, and the like and any combination thereof.

Examples of suitable ethers include cyclic and acyclic ethers such as diethyl ether, diisopropyl ether, n-butyl methyl ether, isobutyl methyl ether, sec-butyl methyl ether, tert-butyl methyl ether, cyclopentyl methyl ether, tetrahydrofuran, 1,4-dioxane, and the like and any combination thereof.

Examples of suitable esters include ethyl acetate, n-propylacetate, isopropyl acetate, tert-butyl acetate, and the like and any combination thereof.

Examples of suitable ketones include acetone, methyl ethyl ketone, methyl isopropyl ketone, methyl isobutyl ketone, cyclopropyl methyl ketone and the like, and any combination thereof.. Examples of suitable nitriles include acetonitrile, benzonitrile, and the like and any combination thereof.

In a preferred embodiment, the inert organic solvent is selected from non-halogenated aliphatic hydrocarbons, non-halogenated cycloaliphatic hydrocarbons, non-halogenated aromatic hydrocarbons, halogenated aliphatic hydrocarbons and any combination thereof.

In a more preferred embodiment, the inert organic solvent is selected from heptane, cyclohexane, cycloheptane, toluene, o-xylene, m-xylene, p-xylene, ethylbenzene, 1,3,5-trimethylbenzene (mesitylene), chlorobenzene, 1,2-dichloroethane, dichloromethane, tetrahydrofuran, dioxane, ethyl acetate, methyl ethyl ketone and benzotrifluoride and any combination thereof.

In an even more preferred embodiment, the inert organic solvent is selected from heptane, cyclohexane, toluene, dichloromethane and any combination thereof.

Still more preferably, the inert organic solvent is selected from heptane, cyclohexane, toluene and any combination thereof.

Particularly preferred inert organic solvents are non-halogenated aromatic hydrocarbons, especially non-halogenated alkylbenzenes which are mono-, di-, or trialkylsubstituted with each alkyl group containing 1 to 3 carbon atoms, and in particular those selected from the group consisting of toluene, o-xylene, m-xylene, p-xylene and any combination thereof. Most preferably, the inert organic solvent is toluene.

The volume ratio of the inert organic solvent to the hydroxyamidine II is generally from 0.01:1 to 20:1, preferably from 0.1:1 to 15:1, more preferably from 0.5:1 to 10:1, and most preferably from 1:1 to 5:1.

In a preferred embodiment of the present invention, the transformation of a compound of formula II with compounds of formula IIa to produce compounds of formula I is carried out in the presence of an organic base.

In one embodiment the base is selected from organic bases such as, for example: tertiary amines, pyridine, substituted pyridines, bicyclic amines and any mixture thereof. Preference is given to tertiary amines, pyridine, substituted pyridines and any mixture thereof. Particular preference is given to pyridine, substituted pyridines and any mixture thereof. Pyridine is especially preferred.

Examples of suitable tertiary amines are tri-(C₁-C₆)-alkylamines such as trimethylamine, triethylamine, tributylamine and diisopropylethylamine; di-(C₁-C₆)-alkyl-phenylamines such as N,N-dimethylaniline and N,N-diethylaniline; N-methyl imidazole, N,N-dimethylaminopyridine and the like.

Examples of suitable substituted pyridines are collidine, lutidines, 2-picoline, 3-picoline, 4-picoline, N,N-dimethyl-4-aminopyridine, 5-ethyl-2-methyl-pyridine and the like.

Examples of suitable bicyclic amines are 1,8-diazabicyclo[5.4.0]undec-7-en, 1,5-diazabicyclo-[4.3.0]non-5-ene, 1,4-diazabicyclo[2.2.2]octane and the like.

In one embodiment the base is an organic base, which is selected from trimethylamine, triethylamine, tributylamine, diisopropylethylamine, N,N-dimethylaniline, N,N-diethylaniline, N-methyl imidazole, pyridine, 2,4,6-collidine, 2,6-lutidine, 2-picoline, 3-picoline, 4-picoline, N,N-dimethyl-4-aminopyridine, 5-ethyl-2-methyl-pyridine,1,8-diazabicyclo[5.4.0]undec-7-en, 1,5-diazabicyclo[4.3.0]non-5-ene, 1,4-diazabicyclo[2.2.2]octane and mixtures thereof.

In another embodiment the base is an organic base, which is selected from trimethylamine, triethylamine, diisopropylethylamine, N,N-dimethylaniline, pyridine, 2,4,6-collidine, 2,6-lutidine, 2-picoline, 3-picoline, 4-picoline, 5-ethyl-2-methyl-pyridine, N,N-dimethylaminopyridine and mixtures thereof.

More preferably the base is an organic base, which is selected from trimethylamine, triethylamine, diisopropylethylamine, pyridine, 2,4,6-collidine, 2,6-lutidine, 2-picoline, 3-picoline, 4-picoline, 5-ethyl-2-methyl-pyridine and mixtures thereof.

In another embodiment, the base is selected from alkali metal and alkaline earth metal phosphates; alkali metal and alkaline earth metal formats; alkali metal and alkaline earth metal acetates; alkali metal and alkaline earth metal carbonates; alkali metal and alkaline earth metal citrates; alkali metal and alkaline earth metal sulfates and any combination thereof, even more preferably selected from alkali metal and alkaline earth metal phosphates; alkali metal and alkaline earth metal formats; alkali metal and alkaline earth metal acetates; alkali metal and alkaline earth metal carbonates; alkali metal and alkaline earth metal citrates and any combination thereof, in particular selected from alkali metal and alkaline earth metal phosphates; alkali metal and alkaline earth metal acetates; particularly sodium acetate, potassium acetate; alkali metal and alkaline earth metal carbonates and any combination thereof, most preferably selected from alkali metal and alkaline earth metal acetates; particularly preferred are sodium carbonate and potassium carbonate.

In another embodiment the base is selected from trimethylamine, triethylamine, tributylamine, diisopropylethylamine, pyridine, 2,4,6-collidine, 2,6-lutidine, 2-picoline, 3-picoline, 4-picoline, 5-ethyl-2-methyl-pyridine, sodium acetate, potassium acetate, sodium carbonate and potassium carbonate and mixtures thereof.

The molar ratio of the base to hydroxyamidine II is generally from 3:1 to 0.4:1 or from 1:1 to 0.2:1, preferably from 0.02:1 to 0.3:1, more preferably from 0.02:1 to 0.25:1, most preferably from 0.02:1 to 0.2:1.

The process of the present invention can be carried out under atmospheric pressure or under elevated or reduced pressure. Typically, the atmospheric and elevated pressure is employed.

In a preferred embodiment the process of the present invention can be carried out at pressure ranges typically from 0.8 atmospheres (atm) to 80 atm, preferably form 1.0 atm to 20 atm, in particular from 1.0 to 7 atm.

The temperature used in the process of the present invention can vary widely and is preferably from 0 to 100°C, 20 to 80°C or 40 to 70°C.

Typical reaction times are in the range of from 1 to 20 hours, preferably from 2 to 15 hours and more preferably from 3 to 10 hours.

In one preferred embodiment, the transformation of a compound of formula II with compounds of formula IIa to produce compounds of formula I is carried out in the presence of an inert organic solvent, wherein the inert organic solvent is selected from trimethylamine, triethylamine, tributylamine, diisopropylethylamine, pyridine, 2,4,6-collidine, 2,6-lutidine, 2-picoline, 3-picoline, 4-picoline, 5-ethyl-2-methyl-pyridine, sodium acetate, potassium acetate, sodium carbonate and potassium carbonate and mixtures thereof; and in the absence of a base, wherein the base is not identical with a compound I, II, Ila, III or an inert organic solvent as defined herein; and at a pressure from 1 to 20 atm; and at a temperature from 0 to 100°C.

In another preferred embodiment, said reaction is carried out in the presence of an inert organic solvent, wherein the inert organic solvent is selected from trimethylamine, triethylamine, tributylamine, diisopropylethylamine, pyridine, 2,4,6-collidine, 2,6-lutidine, 2-picoline, 3-picoline, 4-picoline, 5-ethyl-2-methyl-pyridine, sodium acetate, potassium acetate, sodium carbonate and potassium carbonate and mixtures thereof; and in the absence of a base, wherein the base is not identical with a compound I, II, Ila, III or an inert organic solvent as defined herein; and at a pressure from 1 to 7 atm; and at a temperature from 20 to 80°C.

The process of the present invention may optionally be carried out in the presence of at least one phase-transfer catalyst.

Phase transfer catalysts suitable for use in the process of this invention are those well known in the art such as, for example, quaternary ammonium salts. Examples of suitable phase-transfer catalysts are trimethyl(phenyl) ammonium chloride, bromide, iodide or hydroxide and tetra-n-C₁-C₁₂-alkyl-ammonium chlorides, bromides, iodides or hydroxides, preferably tetra-n-C₁-C₈-alkylammonium chlorides, bromides, iodides or hydroxides, e.g. tetramethylammonium chloride, bromide, iodide or hydroxide, tetraethylammonium chloride, bromide, iodide or hydroxide, tetra-n-propylammonium chloride, bromide, iodide or hydroxide, tetra-n-butylammonium chloride, bromide, iodide or hydroxide, tetra-n-pentylammonium chloride, bromide, iodide or hydroxide, tetra-n-hexylammonium chloride, bromide, iodide or hydroxide, tetra-n-heptylammonium chloride, bromide, iodide or hydroxide, tetra-n-octylammonium chloride, bromide, iodide or hydroxide, methyl-tri-n-butylammonium chloride, bromide, iodide or hydroxide, ethyl-trimethylammonium chloride, bromide, iodide or hydroxide, n-propyl-trimethyl ammonium chloride, bromide, iodide or hydroxide, methyl-triethyl ammonium chloride, bromide, iodide or hydroxide and n-butyl-triethylammonium chloride, bromide, iodide or hydroxide. Of these, the use of tetra-n-C₁-C₄-alkyl-ammonium chlorides, bromides, iodides or hydroxides is preferred, in particular tetra-n-butylammonium chloride, bromide, iodide or hydroxide and methyl-tri-n-butylammonium chloride, bromide, iodide or hydroxide.

The phase-transfer catalysts, which are usually solid in pure form, can be used as such or, preferably, in dissolved form. An effective amount of the phase-transfer catalyst may range from 0.001 to 0.5 molar equivalents, preferably 0.001 to 0.2 molar equivalents relative to compound II.

According to one embodiment of said process, a mixture of the E/Z-isomers of compounds of formula II is used, more preferably said mixture comprises an E/Z-isomer ratio of from 0.1 : 1 to 10 : 1, even more preferably of from 0.5 : 1 to 2 : 1.

According to another embodiment, the E-isomer of compounds of formula II is used.

According to a further embodiment, the Z-isomer of compounds of formula II is used.

The reaction mixture obtained is worked up and the compound I can be isolated in a customary manner, e. g. by an aqueous, extractive workup, in particular extraction with a basic or neutral aqueous medium, and by removing the solvent, e. g. under reduced pressure, or by a combination of these measures. Further purification can be effected, for example, by crystallization, thin-film-evaporation, rectification, sublimation, distillation or by chromatography.

The terms "compounds I", "compounds II", "compounds Ila" and "compounds III" refer to compounds of formulae I, II, Ila and III, respectively. In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "oxo" refers to an oxygen atom =O, which is bound to a carbon atom or sulfur atom, thus forming, for example, a ketonyl -C(=O)- or sulfinyl -S(=O)- group.

The term "formyl" refers to a group C(=O)H.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoro-methyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₄-alkylthio-C₁-C₄-alkyl" refers to alkyl having 1 to 4 carbon atoms (as defined above), wherein one hydrogen atom of the alkyl radical is replaced by a C₁-C₄-alkylthio group.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethyl-imino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms, wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms, wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms, wherein two hydrogen atoms of one carbon atom of the alkyl radical are replaced by a divalent C₂-C₆-alkynyloxyimino radical (C₂-C₆-alkynyl-O-N=).

The term "hydroxyC₁-C₄-alkyl" refers to alkyl having 1 to 4 carbon atoms, wherein one hydrogen atom of the alkyl radical is replaced by a OH group.

The term "aminoC₁-C₄-alkyl" refers to alkyl having 1 to 4 carbon atoms, wherein one hydrogen atom of the alkyl radical is replaced by a NH₂ group.

The term "C₁-C₆-alkylamino" refers to an amino group, which is substituted with one residue independently selected from the group that is defined by the term C₁-C₆-alkyl. Likewise the term "diC₁-C₆-alkylamino" refers to an amino group, which is substituted with two residues independently selected from the group that is defined by the term C₁-C₆-alkyl.

The term "C₁-C₄-alkylamino-C₁-C₄-alkyl" refers to refers to alkyl having 1 to 4 carbon atoms (as defined above), wherein one hydrogen atom of the alkyl radical is replaced by a C₁-C₄-alkyl-NH-group which is bound through the nitrogen. Likewise the term "diC₁-C₄-alkylamino-C₁-C₄-alkyl" refers to refers to alkyl having 1 to 4 carbon atoms (as defined above), wherein one hydrogen atom of the alkyl radical is replaced by a (C₁-C₄-alkyl)₂N- group which is bound through the nitrogen.

The term "aminocarbonyl-C₁-C₄-alkyl" refers to alkyl having 1 to 4 carbon atoms, wherein one hydrogen atom of the alkyl radical is replaced by a -(C=O)-NH₂ group.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₁₁-cycloalkyl" refers to a monocyclic, bicyclic or tricyclic saturated univalent hydrocarbon radical having 3 to 11 carbon ring members that is connected through one of the ring carbon atoms by substitution of one hydrogen atom, such as cyclopropyl (C₃H₅), cyclobutyl, cyclopentyl, cyclohexyl, cycloheptyl, cyclooctyl, bicyclo[1.1.0]butyl, bicyclo[2.1.0]pentyl, bicy-clo[1.1.1]pentyl, bicyclo[3.1.0]hexyl, bicyclo[2.1.1]hexyl, norcaranyl (bicyclo[4.1.0]heptyl) and norbornyl (bicyclo[2.2.1]heptyl). Further examples of bicyclic or tricyclic cycloalkyl radicals are found herein as examples R¹.1 to R¹.57.

The term "C₃-C₁₁-cycloalkyl" refers to a monocyclic, bicyclic or tricyclic saturated univalent hydrocarbon radical having 3 to 11 carbon ring members that is connected through one of the ring carbon atoms by substitution of one hydrogen atom, such as cyclopropyl (C₃H₅), cyclobutyl, cyclopentyl, cyclohexyl, cycloheptyl, cyclooctyl, bicyclo[1.1.0]butyl, bicyclo[2.1.0]pentyl, bicy-clo[1.1.1]pentyl, bicyclo[3.1.0]hexyl, bicyclo[2.1.1]hexyl, norcaranyl (bicyclo[4.1.0]heptyl) and norbornyl (bicyclo[2.2.1]heptyl).

The term "C₃-C₁₁-cycloalkyl-C₁-C₆-alkyl" refers to alkyl having 1 to 11 carbon atoms, wherein one hydrogen atom of the alkyl radical is replaced by a C₃-C₁₁-cycloalkyl group as defined above.

The term "C₃-C₁₁-cycloalkoxy" refers to a cyclic univalent hydrocarbon radical having 3 to 11 carbon ring members (as defined above) that is bonded via an oxygen, at any position in the cycloalkyl group, for example cyclopropyloxy.

The terms "-C(=O)-C₁-C₄-alkyl", "-C(=O)-C₁-C₄-alkoxy" and "-C(=O)-C₃-C₁₁-cycloalkyl" refer to radicals which are attached through the carbon atom of the -C(=O)- group.

The term "aliphatic" refers to compounds or radicals composed of carbon and hydrogen and which are non-aromatic compounds. An "alicyclic" compound or radical is an organic compound that is both aliphatic and cyclic. They contain one or more all-carbon rings which may be either saturated or unsaturated, but do not have aromatic character.

The terms "cyclic moiety" or "cyclic group" refer to a radical which is an alicyclic ring or an aromatic ring, such as, for example, phenyl or heteroaryl.

The term "and wherein any of the aliphatic or cyclic groups are unsubstituted or substituted with..." refers to aliphatic groups, cyclic groups and groups, which contain an aliphatic and a cyclic moiety in one group, such as in, for example, C₃-C₈-cycloalkyl-C₁-C₄-alkyl; therefore a group which contains an aliphatic and a cyclic moiety both of these moieties may be substituted or unsubstituted independently of each other.

The term "phenyl" refers to an aromatic ring systems incuding six carbon atoms (commonly referred to as benzene ring.

The term "heteroaryl" refers to aromatic monocyclic or polycyclic ring systems incuding besides carbon atoms, 1, 2, 3 or 4 heteroatoms independently selected from the group consisting of N, O and S.

The term "saturated 3- to 7-membered carbocycle" is to be understood as meaning monocyclic saturated carbocycles having 3, 4 or 5 carbon ring members. Examples include cyclopropyl, cyclopentyl, cyclohexyl, cycloheptyl, and the like.

The term "3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms", is to be understood as meaning both, aromatic mono- and bicyclic heteroaromatic ring systems, and also saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azet-idine, thiethane, [1,2]dioxetane, [1,2]dithietane, [1,2]diazetidine;
and a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetra-hydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

In respect of the variables, the embodiments of the intermediates correspond to the embodiments of the compounds I. Preference is given to those compounds I and, where applicable, also to compounds of all subformulae provided herein, e. g. formulae I.1, I.2, II, la, III, wherein the variables have independently of each other or more preferably in combination (any possible combination of 2 or more substituents as defined herein) the following meanings:
According to a further embodiment, W in compounds of formulae I, II and III is O.

Further embodiments of the present invention relate to the process for preparing compounds I, wherein group R¹ in formulae of compounds I, II and III is defined as follows:
Embodiment 1.1: R¹ is C₁-C₆-alkyl, C₂-C₆-alkenyl, C₂-C₆-alkynyl, C₃-C₈-cycloalkyl, C₃-C₈-cycloalkenyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms; and wherein any of the aliphatic or cyclic groups are unsubstituted or substituted with 1, 2, 3 or up to the maximum possible number of identical or different radicals R^{1a} as defined or preferably defined herein.
Embodiment 1.2: R¹ is phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms; and wherein any of the cyclic groups are unsubstituted or substituted with 1, 2, 3 or up to the maximum possible number of identical or different radicals R^{1a} as defined or preferably defined herein.
Embodiment 1.3: R¹ is C₃-C₈-cycloalkyl or C₃-C₈-cycloalkenyl; and wherein the cyclic group is unsubstituted or substituted with 1, 2, 3 or up to the maximum possible number of identical or different radicals R^{1a} as defined or preferably defined herein.
Embodiment 1.4: R¹ is C₁-C₆-alkyl; and wherein the alkyl group is unsubstituted or substituted with 1, 2, 3 or up to the maximum possible number of identical or different radicals R^{1a} as defined or preferably defined herein.
Embodiment 1.5: R¹ is difluoromethyl, trifluoromethyl, 2,2-difluoroethyl, 2,2,2-trifluoroethyl, 2-chloro-2-fluoroethyl, 2-chloro-2,2-difluoroethyl, 2,2,2-trichloroethyl and pentafluoroethyl, 3,3,3-trifluoropropyl, CH₂CF₂CF₃ or CF₂CF₂CFs, CH(CH₃)CF₃, CH₂CF₂CH₃, CH₂C(CH₃)₂F, CH₂CH(CH₃)CF₃ or CH₂C(CH₃)₂CF₃.

Further embodiments of the present invention relate to the process for preparing compounds I, wherein group R^{1a} is defined as follows:
In one embodiment R^{1a} is selected from the group consisting of halogen, C₁-C₆-alkyl and C₃-C₈-cycloalkyl; particularly from methyl, ethyl, fluorine and chlorine; more particularly from fluorine and chlorine.

In another embodiment R² in formulae of compounds I, II and III is defined as follows:
Embodiment 2.1: R² independently of each other are hydrogen, formyl, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, C₁-C₆-alkylamino or diC₁-C₆-alkylamino; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy.
Embodiment 2.2: R² independently of each other are hydrogen, formyl, methyl, ethyl, n-propyl, iso-propyl, methoxy, ethyoxy, propyloxy, cyclopropyl, cyclopropyl-CH₂-, allyl, phenyl, 4-F-phenyl, 2-F-phenyl, C₁-C₆-alkylamino or diC₁-C₆-alkylamino.
Embodiment 2.3: R² independently of each other are hydrogen, formyl, C₁-C₆-alkyl, C₁-C₆-alkoxy, C₂-C₆-alkenyl, propargyl, C₃-C₈-cycloalkyl, C₁-C₆-alkylamino or diC₁-C₆-alkylamino.
Embodiment 2.4: R² independently of each other are hydrogen, formyl, methyl, ethyl, n-propyl, iso-propyl, methoxy, ethyoxy, propyloxy, cyclopropyl, cyclopropyl-CH₂-, allyl, C₁-C₆-alkylamino or diC₁-C₆-alkylamino.
Embodiment 2.5: R² independently of each other are hydrogen, C₁-C₆-alkyl, C₁-C₆-alkoxy, C₂-C₆-alkenyl or propargyl, C₁-C₆-alkylamino or diC₁-C₆-alkylamino.
Embodiment 2.6: R² independently of each other are hydrogen, methy, ethyl, methoxy, ethyoxy, propyloxy, C₁-C₆-alkylamino or diC₁-C₆-alkylamino.
Embodiment 2.7: R² is hydrogen.

According to a further embodiment in compounds of formulae I, II, and III, R² is C₁-C₄-alkyl, C₁-C₆-alkoxy or C₃-C₈-cycloalkyl, and wherein any of the aliphatic or cyclic groups in R² are unsubstituted or substituted with 1, 2, 3 or up to the maximum possible number of identical or different radicals selected from halogen.

In another aspect R² in formulae of compounds I, II and III is hydrogen, formyl, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, C₁-C₆-alkylamino or diC₁-C₆-alkylamino; and R¹ in formulae of compounds I, II and III is C₁-C₆-alkyl, C₃-C₈-cycloalkyl, C₃-C₈-cycloalkenyl, C₂-C₆-alkenyl, C₂-C₆-alkynyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms; and wherein any of the aliphatic or cyclic groups in R¹ are unsubstituted or substituted with 1, 2, 3, 4 or up to the maximum possible number of identical or different radicals R^{1a} as defined or preferably defined herein.

In another aspect R² in formulae of compounds I, II and III is hydrogen, formyl, methyl, ethyl, n-propyl, iso-propyl, methoxy, ethyoxy, propyloxy, cyclopropyl, cyclopropyl-CH₂-, allyl, C₁-C₆-alkylamino or diC₁-C₆-alkylamino; and R¹ in formulae of compounds I, II and III is C₁-C₆-alkyl, C₃-C₈-cycloalkyl, C₃-C₈-cycloalkenyl, C₂-C₆-alkenyl or C₂-C₆-alkynyl; and wherein any of the aliphatic or cyclic groups in R¹ are unsubstituted or substituted with 1, 2, 3, 4 or up to the maximum possible number of identical or different radicals selected from the group consisting of halogen or C₁-C₆-alkyl.

Further embodiments with regard to the meaning of the variables in compounds of formula I are as follows:
Embodiment E1: A¹ and A² are CH and R^{A} is hydrogen.
Embodiment E2: A¹ and A² are CH and R^{A} is fluorine.

In one embodiment the present invention relates to a process for preparing compounds of formula I, wherein the combination of variables A¹, A², R^{A} corresponds to any one of the Embodiments E1, E2 defined above; and
- R: is -COOH, -COOR¹ or -C(=W)NR¹R²;
- W: is O or S;
- R¹: is methyl, ethyl, *n*-propyl, *iso*-propyl, *n*-butyl, *sec*-butyl, *iso*-butyl, 2-methoxyiminoethyl, cyclopropyl, bicyclo[1.1.1]pentan-1-yl, or phenyl; and wherein the phenyl group is unsubstituted or substituted with 1, 2, 3 or up to the maximum possible number of identical or different radicals selected from the group consisting of fluorine, chlorine, cyano, methyl, ethyl, methoxy, trifluoromethyl, trifluoromethoxy, difluoromethyl, difluoromethoxy and cyclopropyl.
- R²: is hydrogen, methyl or ethyl.

In one embodiment the present invention relates to a process for preparing compounds of formula I, wherein the combination of variables A¹, A², R^{A} corresponds to any one of the Embodiments E1, E2 defined above; and
- R: is -COOH, -COOR¹ or -C(=W)NR¹R²;
- W: is O or S;
- R¹: is methyl, ethyl, *n*-propyl, *iso*-propyl, *n*-butyl, *sec*-butyl, *iso*-butyl, 2-methoxyiminoethyl, cyclopropyl, bicyclo[1.1.1]pentan-1-yl, or phenyl; and wherein the phenyl group is unsubstituted or substituted with 1 radical selected from the group consisting of fluorine and 2-difluoromethoxy-phenyl;
- R²: is hydrogen, methyl or ethyl.

In one embodiment the present invention relates to a process for preparing compounds of formula I, wherein the combination of variables A¹, A², R^{A} corresponds to any one of the Embodiments E1, E2 defined above; and
- R: is -C(=O)NR¹R²;
- R¹: is methyl, 2-methoxyiminoethyl, bicyclo[1.1.1]pentan-1-yl, 2-fluoro-phenyl, 4-fluoro-phenyl, or 2-difluoromethoxy-phenyl;
- R²: is hydrogen.

In one embodiment the present invention relates to a process for preparing compounds of formula I, wherein the combination of variables A¹, A², R^{A} corresponds to any one of the Embodiments E1, E2 defined above; and
- R: is -C(=S)NR¹R²;
- R¹: is methyl, 2-methoxyiminoethyl, bicyclo[1.1.1]pentan-1-yl, 2-fluoro-phenyl, 4-fluoro-phenyl, or 2-difluoromethoxy-phenyl;
- R²: is hydrogen.

In a further embodiment the present invention relates to a process for preparing compounds of formula I, wherein
- A¹ and A²: are CH;
- R^{A}: is hydrogen;
- R: is -C(=O)NR¹R²;
- R¹: is methyl, 2-methoxyiminoethyl, bicyclo[1.1.1]pentan-1-yl, 2-fluoro-phenyl, 4-fluoro-phenyl, or 2-difluoromethoxy-phenyl;
- R²: is hydrogen.

In a further embodiment the present invention relates to a process for preparing compounds of formula I, wherein
- A¹ and A²: are CH;
- R^{A}: is hydrogen;
- R: is -C(=S)NR¹R²;
- R¹: is methyl, 2-methoxyiminoethyl, bicyclo[1.1.1]pentan-1-yl, 2-fluoro-phenyl, 4-fluoro-phenyl, or 2-difluoromethoxy-phenyl;
- R²: is hydrogen.

The invention is illustrated by the following examples:

### Example 1: Preparation of N-methyl-N-[[4-[5-(trifluoromethyl)-1,2,4-oxadiazol-3-yl]phenyl]methyl]cyclopropanecarboxamide

5.0 g (17.6 mmol) *N*-[[4-[(*Z*)-*N*'-hydroxycarbamimidoyl]phenyl]methyl]-*N*-methyl-cyclopropanecarboxamide and 25 g (271 mmol) toluene were placed in a glass reactor and cooled to -22°C. After that 4.6 g (35 mmol) trifluoroacetyl chloride was dosed continuously under vigorous stirring over 10 minutes at -22°C to the reaction mixture. The reaction mixture was stirred for further 45 minutes at -15°C to -13°C. Then the reaction mixture was heated up to 25°C. 25 g water was added to the reaction mixture. After phase separation the organic phase was extracted with 20 g water. The solvent of the organic phase was distilled off under reduced pressure (40°C; 2 kPa). Residue: 3.1 g; HPLC-purity of 76.6%; Yield: 42%.

### Example 2: Preparation of 2,2-difluoro-N-methyl-2-[4-[5-(trifluoromethyl)-1,2,4-oxadiazol-3-yl]phenyl]acetamide

20.0 g (78 mmol) 2,2-difluoro-2-[4-[(*Z*)-*N*'-hydroxycarbamimidoyl]phenyl]-N-methyl-acetamide and 200 mL dichloromethane were placed in a pressure reactor and cooled to -10°C. Then 21.5 g (162 mmol) trifluoroacetyl chloride was dosed continuously under vigorous stirring over 10 minutes at -10°C to the reaction mixture. The reaction mixture was stirred for 4 hours at 100°C and 990 kPa. Then, after the reactor was cooled down to 25°C, the pressure of the reactor was released and 200 g water was added to the reaction mixture. After phase separation the organic phase was extracted once with 200 g of an aqueous solution of sodium hydroxide (5 % by weight) and once with 100g water. The organic phase was dried over magnesium sulfate. The solvent was the removed under reduced pressure (40°C; 2 kPa). Residue: 17.5 g; HPLC gave a purity of 76.3%; Yield: 66.3%.

### Example 3: Preparation of 3-(p-tolyl)-5-(trifluoromethyl)-1,2,4-oxadiazole

100 g (0.67 mol) *N*'-hydroxy-4-methyl-benzamidine were suspended in 450 g (5.22 mol) tetra-hydro-2-methylfuran (Me-THF). In total, 203 g (1.53 mol) trifluoroacetyl chloride were dosed in between 0°C and 25°C in 5 hours. Then, the volatiles were removed *in vacuo* (60°C, 250 to 5 mbar) to yield 138 g (>99 % HPLC purity, 91 % yield) 3-(*p*-tolyl)-5-(trifluoromethyl)-1,2,4-oxadiazole.

### Example 4: Preparation of 3-(p-tolyl)-5-(trifluoromethyl)-1,2,4-oxadiazole

100 g (0.67 mol) *N*'-hydroxy-4-methyl-benzamidine were suspended in 450 g (5.22 mol) Me-THF. In total, 203 g (1.53 mol) trifluoroacetylchloride were dosed 40°C in 3 hours. The volatiles were removed *in vacuo* (60°C, 10 to 0.5 kPa) to yield 137 g (>98 % HPLC purity, 90 % yield) 3-(*p*-tolyl)-5-(trifluoromethyl)-1,2,4-oxadiazole.

### Example 5: Preparation of N-methyl-4-[5-(trifluoromethyl)-1,2,4-oxadiazol-3-yl]benzamide - Solvent screening

0.1 g (0.517 mmol) *N*'-hydroxy-4-methyl-benzamidine were suspended in 5 g solvent. Then, 1.13-1.26 g (0.0566 mol) ca. 10-12 % solution of trifluoroacetyl chloride in Me-THF or ethyl acetate were added at room temperature and stirred for 1 hour. The table below shows the HPLC % of *N*-methyl-4-[5-(trifluoromethyl)-1,2,4-oxadiazol-3-yl]benzamide following this procedure and in the presence of several different organic solvents.

| Solvent | HPLC% |
|---|---|
| *N,N*-dimethylformamide | 85 |
| NMP | 50 |
| Tetrahydro-2-methylfuran | 96 |
| Acetonitrile | 87 |
| Tetrahydrofurane | 92 |
| Methyl *tert*-butylether | 35 |
| Ethyl acetate | 85 |
| Me-Imidazolin | 99 |
| Acetone | 44 |
| Dioxane | 73 |
| DME | 96 |
| Dichloromethane | 41 |

Example 6: Preparation of *N*-methyl-4-[5-(trifluoromethyl)-1,2,4-oxadiazol-3-yl]benzamide 5 g (0.026 mol) *N*'-hydroxy-4-methyl-benzamidine were suspended in 20 g (0.232 mol) in Me-THF. 62 g (0.0566 mol) ca. 12% solution of trifluoroacetyl chloride in Me-THF were added at room temperature and stirred for 102 hours. After addition of 140 g of a 4% sodium bicarbonate-solution, phases were separated. The organic phase was washed with water and all volatiles were removed *in vacuo* (up to 80°C and 0.5 kPa) to yield 6.6 g (>98 % HPLC purity, 93 % yield) *N*-methyl-4-[5-(trifluoromethyl)-1,2,4-oxadiazol-3-yl]benzamide.

### Example 7: Preparation of N-(2-fluorophenyl)-4-[5-(trifluoromethyl)-1,2,4-oxadiazol-3-yl]benzamide

*N*-(2-fluorophenyl)-4-[(*Z*)-*N*'-hydroxycarbamimidoyl]benzamide (1000 mg, 3.65 mmol) was suspended in Me-THF (10 mL) and trifluoroacetyl chloride (12% solution in in Me-THF; 1.9 g, 14.63 mmol) was added dropwise at room temperature and stirred for 10 hours at 60°C. The reaction mass was quenched with water following by adjusting pH to 8 with a 4% sodium bicarbonate solution. The organic phase was separated, washed with water and all volatiles were removed *in vacuo* (up to 80°C and 0.5 kPa) to yield 955 mg of the title product.

Example 8: Preparation of 4-[5-(trifluoromethyl)-1,2,4-oxadiazol-3-yl]benzoic acid 4-[(*Z*)-*N*'-hydroxycarbamimidoyl]benzoic acid (500 mg, 3 mmol) was suspended in Me-THF (2 mL) and trifluoroacetyl chloride (12% solution in in Me-THF; 6.2 g, 6 mmol) was added dropwise at room temperature and stirred for 20 hours. The reaction mass was quenched with water following by adjusting pH to 8 with a 4% sodium bicarbonate solution. The organic phase was separated, washed with water and all volatiles were removed *in vacuo* (up to 80°C and 0.5 kPa) to yield the title product.

### Example 9: Preparation of N-methyl-N-[[4-[5-(trifluoromethyl)-1,2,4-oxadiazol-3-yl]phenyl]methyl]methanesulfonamide

*N*'-hydroxy-4-[[methyl(methylsulfonyl)amino]methyl]benzamidine (500 mg, 2 mmol) was suspended Me-THF (2 mL) and trifluoroacetyl chloride (12% solution in in Me-THF; 16.2 g, 14.63 mmol) was added dropwise at room temperature and stirred for 10 hours at 60°C. The reaction mass was quenched with water following by adjusting pH to 8 with a 4% sodium bicarbonate solution. The organic phase was separated, washed with water and all volatiles were removed *in vacuo* (up to 80°C and 0.5 kPa) to yield the title product.

### Example 10: Preparation of N-methyl-N-[[4-[5-(trifluoromethyl)-1,2,4-oxadiazol-3-yl]phenyl]methyl]cyclopropanecarboxamide

*N*-[[4-[(Z)-N'-hydroxycarbamimidoyl]phenyl]methyl]-N-methyl-cyclopropanecarboxamide (500 mg, 2 mmol) was suspended in Me-THF (2 mL) and trifluoroacetyl chloride (12% solution in in Me-THF; 6.2 g, 6 mmol) was added dropwise at room temperature and stirred for 20 hours. The reaction mass was quenched with water following by adjusting pH to 8 with a 4% sodium bicarbonate solution. The organic phase was separated, washed with water and all volatiles were removed *in vacuo* (up to 80°C and 0.5 kPa) to yield the title product.

## Claims

1. A process for preparing compounds of formula I, 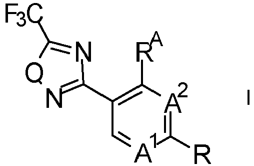 wherein
A¹ is CH;
A² is CH;
R^{A} is hydrogen or fluorine;
R is -COOH, -COOR¹ or -C(=W)NR¹R²;
W is O or S;
R¹ is C₁-C₆-alkyl, C₂-C₆-alkenyl, C₂-C₆-alkynyl, C₁-C₆-alkoxyimino-C₁-C₄-alkyl, C₃-C₁₁-cycloalkyl, C₃-C₈-cycloalkenyl, phenyl-C₁-C₄-alkyl, heteroaryl-C₁-C₄-alkyl, phenyl or heteroaryl; and wherein the heteroaryl group is a 5- or 6-membered aromatic heterocycle, wherein the ring includes besides carbon atoms 1, 2, 3 or 4 heteroatoms selected from N, O and S as ring member atoms; and wherein any of the aliphatic or cyclic groups are unsubstituted or substituted with 1, 2, 3 or up to the maximum possible number of identical or different radicals selected from the group consisting of halogen, cyano, C₁-C₆-alkyl and C₁-C₆-alkoxy;
R² is hydrogen, formyl, C₁-C₆-alkyl, C₁-C₆-alkoxy, C₂-C₆-alkenyl, propargyl, C₃-C₈-cycloalkyl, C₃-C₈-cycloalkenyl, C₃-C₈-cycloalkyl-C₁-C₄-alkyl, phenyl, C₁-C₆-alkylamino or diC₁-C₆-alkylamino; and wherein any of the aliphatic or cyclic groups are unsubstituted or substituted with 1, 2, 3, 4 or up to the maximum possible number of identical or different radicals selected from the group consisting of halogen, cyano, C₁-C₆-alkyl and C₁-C₆-alkoxy;
the process comprising:
reacting a compound of formula II, 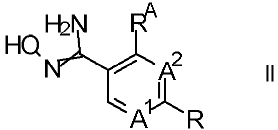
wherein A¹, A², R^{A} and R is as defined above, with a trifluoroacetyl halide of formula IIa, 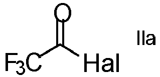
wherein Hal is chlorine or fluorine; and wherein the process is conducted in the presence of an inert organic solvent; and wherein the process is conducted at a temperature from 0°C to 100°C.

2. The process as claimed in claim 1, wherein the process is conducted in the presence of an inert organic solvent selected from non-halogenated aliphatic hydrocarbons, non-halogenated cycloaliphatic hydrocarbons, non-halogenated aromatic hydrocarbons, halogenated aliphatic hydrocarbons, halogenated aromatic hydrocarbons, amides, ethers, esters, ketones, nitriles and any combination thereof.

3. The process as claimed in claim 1 or 2, wherein Hal in compound IIa is chlorine.

4. The process as claimed claim 1, wherein the process is conducted in the presence of a base selected from trimethylamine, triethylamine, tributylamine, diisopropylethylamine, pyridine, 2,4,6-collidine, 2,6-lutidine, 2-picoline, 3-picoline, 4-picoline, 5-ethyl-2-methylpyridine, sodium acetate, potassium acetate, sodium carbonate, potassium carbonate and mixtures thereof.

5. The process as claimed in any one of claims 1 to 4, wherein the process is conducted at a pressure from 1 atm to 20 atm.

6. The process as claimed in any one of claims 1 to 5, wherein the process is conducted at a temperature from 20°C to 80°C.

7. The process as claimed in any one of the claims 1 to 6, the process comprising two steps:
i) reacting a compound of formula V, 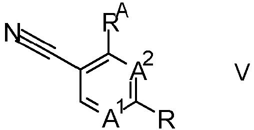 wherein R is as defined in claim 1, with hydroxylamine or a salt thereof;
ii) reacting the product of step i), a compound of formula II, 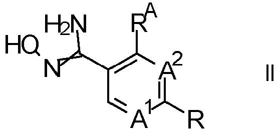 wherein R is as defined above, with a trifluoroacetyl halide according to the process as defined in any one of claims 1 to 6.

## Patentansprüche

1. Verfahren zur Herstellung von Verbindungen der Formel I, 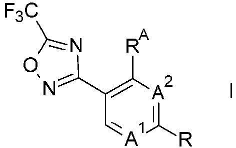 wobei
A¹ CH ist;
A² CH ist;
RA Wasserstoff oder Fluor ist;
R -COOH, -COOR¹ oder -C(=W)NR¹R² ist;
W O oder S ist;
R¹ C₁-C₆-Alkyl, C₂-C₆-Alkenyl, C₂-C₆-Alkinyl, C₁-C₆-Alkoxyimino-C₁-C₄-alkyl, C₃-C₁₁-Cycloalkyl, C₃-C₈-Cycloalkenyl, Phenyl-C₁-C₄-alkyl, Heteroaryl-C₁-C₄-alkyl, Phenyl oder Heteroaryl ist; und wobei die Heteroarylgruppe ein 5- oder 6-gliedriger aromatischer Heterocyclus ist, wobei der Ring neben Kohlenstoffatomen 1, 2, 3 oder 4 Heteroatome, ausgewählt aus N, O und S, als Ringgliedatome enthält; und wobei jede der aliphatischen oder cyclischen Gruppen unsubstituiert oder mit 1, 2, 3 oder bis zur maximal möglichen Anzahl gleicher oder verschiedener Reste substituiert ist, die aus der Gruppe ausgewählt sind, bestehend aus Halogen, Cyano, C₁-C₆-Alkyl und C₁-C₆-Alkoxy;
R² Wasserstoff, Formyl, C₁-C₆-Alkyl, C₁-C₆-Alkoxy, C₂-C₆-Alkenyl, Propargyl, C₃-C₈-Cycloalkyl, C₃-C₈-Cycloalkenyl, C₃-C₈-Cycloalkyl-C₁-C₄-alkyl, Phenyl, C₁-C₆-Alkylamino oder Di-C₁-C₆-alkylamino ist; und wobei jede der aliphatischen oder cyclischen Gruppen unsubstituiert oder mit 1, 2, 3, 4 oder bis zur maximal möglichen Anzahl gleicher oder verschiedener Reste substituiert ist, die aus der Gruppe ausgewählt sind, bestehend aus Halogen, Cyano, C₁-C₆-Alkyl und C₁-C₆-Alkoxy;
wobei das Verfahren umfasst:
Umsetzen einer Verbindung der Formel II, 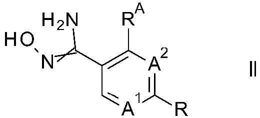
wobei A¹, A², RA und R wie vorstehend definiert sind, mit einem Trifluoracetylhalogenid der Formel Ila, 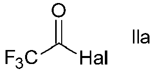
wobei Hal Chlor oder Fluor ist; und wobei das Verfahren in Gegenwart eines inerten organischen Lösungsmittels durchgeführt wird; und wobei das Verfahren bei einer Temperatur von 0°C bis 100°C durchgeführt wird.

2. Verfahren nach Anspruch 1, wobei das Verfahren in Gegenwart von nicht halogenierten aliphatischen Kohlenwasserstoffen, nicht halogenierten cycloaliphatischen Kohlenwasserstoffen, nicht halogenierten aromatischen Kohlenwasserstoffen, halogenierten aliphatischen Kohlenwasserstoffen, halogenierten aromatischen Kohlenwasserstoffen, Amiden, Ethern, Estern, Ketonen, Nitrilen und einer beliebigen Kombination davon durchgeführt wird.

3. Verfahren nach Anspruch 1 oder 2, wobei Hal in Verbindung IIa Chlor ist.

4. Verfahren nach Anspruch 1, wobei das Verfahren in Gegenwart einer Base durchgeführt wird, die aus Trimethylamin, Triethylamin, Tributylamin, Diisopropylethylamin, Pyridin, 2,4,6-Collidin, 2,6-Lutidin, 2-Picolin, 3-Picolin, 4-Picolin, 5-Ethyl-2-methylpyridin, Natriumacetat, Kaliumacetat, Natriumcarbonat, Kaliumcarbonat und Gemischen davon ausgewählt ist.

5. Verfahren nach einem der Ansprüche 1 bis 4, wobei das Verfahren bei einem Druck von 1 atm bis 20 atm durchgeführt wird.

6. Verfahren nach einem der Ansprüche 1 bis 5, wobei das Verfahren bei einer Temperatur von 20°C bis 80°C durchgeführt wird.

7. Verfahren nach einem der Ansprüche 1 bis 6, wobei das Verfahren zwei Schritte umfasst:
i) Umsetzen einer Verbindung der Formel V, 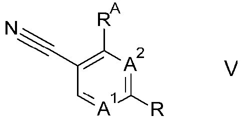 wobei R wie in Anspruch 1 definiert ist, mit Hydroxylamin oder einem Salz davon;
ii) Umsetzen des Produkts aus Schritt i), einer Verbindung der Formel II, 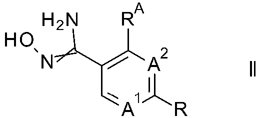 wobei R wie vorstehend definiert ist, mit einem Trifluoracetylhalogenid gemäß dem Verfahren, wie es in einem der Ansprüche 1 bis 6 definiert ist.

## Revendications

1. Procédé de préparation de composés de formule I, 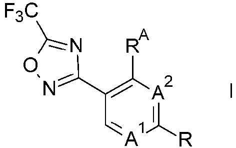 dans lequel
A¹ est CH ;
A² est CH ;
RA est hydrogène ou fluor ;
R est -COOH, -COOR¹ ou -C(=W)NR¹R² ;
W est O ou S ;
R¹ est alkyle en C₁-C₆, alcényle en C₂-C₆, alcynyle en C₂-C₆, alkoxyimino en C₁-C₆-alkyle en C₁-C₄, cycloalkyle en C₃-C₁₁, cycloalcényle en C₃-C₈, phényl-alkyle en C₁-C₄, hétéroaryl-alkyle en C₁-C₄, phényle ou hétéroaryle ; et dans lequel le groupe hétéroaryle est un hétérocycle aromatique à 5 ou 6 chaînons, dans lequel le cycle comprend, outre des atomes de carbone, 1, 2, 3 ou 4 hétéroatomes choisis parmi N, O et S en tant qu'atomes membres du cycle ; et dans lequel l'un quelconque des groupes aliphatiques ou cycliques est non substitué ou substitué par 1, 2, 3 ou jusqu'au nombre maximal possible de radicaux identiques ou différents choisis dans le groupe constitué par halogène, cyano, alkyle en C₁-C₆ et alcoxy en C₁-C₆ ;
R² est hydrogène, formyle, alkyle en C₁-C₆, alcoxy en C₁-C₆, alcényle en C₂-C₆, propargyle, cycloalkyle en C₃-C₈, cycloalcényle en C₃-C₈, cycloalkyle en C₃-C₈-alkyle en C₁-C₄, phényle, alkylamino en C₁-C₆ ou di(alkyle en C₁-C₆)amino ; et dans lequel l'un quelconque des groupes aliphatiques ou cycliques est non substitué ou substitué par 1, 2, 3, 4 ou jusqu'au nombre maximal possible de radicaux identiques ou différents choisis dans le groupe constitué par halogène, cyano, alkyle en C₁-C₆ et alcoxy en C₁-C₆ ;
le procédé comprenant :
la mise en réaction d'un composé de formule II, 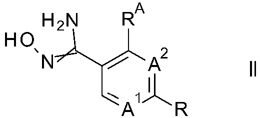
dans lequel A¹, A², RA et R sont tels que définis ci-dessus, avec un halogénure de trifluoroacétyle de formule IIa, 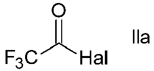
dans lequel Hal est chlore ou fluor ; et dans lequel le procédé est mis en œuvre en présence d'un solvant organique inerte ; et dans lequel le procédé est mis en œuvre à une température de 0°C à 100°C.

2. Procédé selon la revendication 1, dans lequel le procédé est mis en œuvre en présence d'hydrocarbures aliphatiques non halogénés, d'hydrocarbures cycloaliphatiques non halogénés, d'hydrocarbures aromatiques non halogénés, d'hydrocarbures aliphatiques halogénés, d'hydrocarbures aromatiques halogénés, d'amides, d'éthers, d'esters, de cétones, de nitriles et de toute combinaison de ceux-ci.

3. Procédé selon la revendication 1 ou 2, dans lequel Hal dans le composé IIa est chlore.

4. Procédé selon la revendication 1, dans lequel le procédé est mis en œuvre en présence d'une base choisie parmi la triméthylamine, la triéthylamine, la tributylamine, la diiso-propyléthylamine, la pyridine, la 2,4,6-collidine, la 2,6-lutidine, la 2-picoline, la 3-picoline, la 4-picoline, la 5-éthyl-2-méthylpyridine, l'acétate de sodium, l'acétate de potassium, le carbonate de sodium, le carbonate de potassium et leurs mélanges.

5. Procédé selon l'une quelconque des revendications 1 à 4, dans lequel le procédé est mis en œuvre à une pression de 1 atm à 20 atm.

6. Procédé selon l'une quelconque des revendications 1 à 5, dans lequel le procédé est mis en œuvre à une température de 20°C à 80°C.

7. Procédé selon l'une quelconque des revendications 1 à 6, le procédé comprenant deux étapes :
i) la mise en réaction d'un composé de formule V, 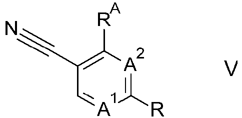 dans lequel R est tel que défini dans la revendication 1, avec de l'hydroxylamine ou un sel de celle-ci ;
ii) la mise en réaction du produit de l'étape i), un composé de formule II, 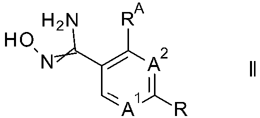 dans lequel R est tel que défini ci-dessus, avec un halogénure de trifluoroacétyle selon le procédé tel que défini dans l'une quelconque des revendications 1 à 6.
